# EUROPÄISCHE PATENTSCHRIFT

(11) **EP 1 762 194 B1**
(45) Veröffentlichungstag und Bekanntmachung des Hinweises auf die Patenterteilung: **02.04.2008**
(21) Anmeldenummer: 06018916.4
(22) Anmeldetag: 09.09.2006
(51) Int. Cl.: A61B 17/34

(54) **Ventileinrichtung für medizinische Instrumente**
Valve assembly for medical instruments
Dispositif de soupape pour instruments medicaux

(30) Priorität: 09.09.2005 DE 102005042892
(43) Veröffentlichungstag der Anmeldung: 14.03.2007
(73) Patentinhaber: Karl Storz GmbH & Co. KG, 78532 Tuttlingen (DE)
(72) Erfinder: Sauer, Michael, 78532 Tuttlingen (DE); Oberländer, Martin, 78234 Engen (DE); Teichtmann, Elmar, 78054 Villingen-Schwenningen (DE)
(74) Vertreter: Hofmeister, Frank

(56) Entgegenhaltungen:
- EP-A1- 1 481 641
- WO-A-03/041598
- DE-U1- 29 619 635

## Beschreibung

Die Erfindung betrifft eine Ventileinrichtung für medizinische Instrumente, insbesondere Trokare, mit einem als Klappe ausgebildeten Ventilkörper zum Öffnen und Schließen eines Instrumenten- und/oder Strömungskanals, der verschwenkbar auf einer in einem Gehäuse gelagerten Schwenkachse angeordnet ist.

Derartige Ventileinrichtungen für medizinische Instrumente werden beispielsweise bei Trokaren verwendet. Trokare dienen dazu, Operationsinstrumente für endoskopische Operationen beispielsweise in die Bauchhöhle eines Patienten einzuführen. Dazu wird die Trokarhülse an die Bauchdecke angesetzt, ein Trokardorn in den hohlen Instrumentenkanal eingeschoben, mit Hilfe des Trokardorns eine Öffnung in die Bauchdecke gestoßen und anschließend die Trokarhülse durch die Öffnung in den Bauchraum eingeführt. Der Trokardorn kann dann wieder aus der Trokarhülse herausgezogen werden. Da es bei endoskopischen Operationen des Bauchraums üblich ist, den Bauchraum zur Erweiterung des Operationsraums und Ausbildung eines Pneumoperitoneums mit Gas zu befüllen, ist der hohle Instrumentenkanal der Trokarhülse über einen Ventilkörper verschließbar, damit das Gas bei entnommenem Instrument nicht über die Trokarhülse aus dem Bauchraum entweichen kann. Die bekannten Ventilkörper sind dabei so ausgebildet, dass sie durch ein in den Instrumentenkanal eingeschobenes Instrument geöffnet werden und sich beim Herausziehen des Instruments auch selbsttätig wieder schließen.

Eine gattungsgemäße Ventileinrichtung für ein medizinisches Instrument, nämlich ein Trokar, ist beispielsweise aus der DE 297 00 762 U1 bekannt. Bei diesem bekannten Trokar ist die den Ventilkörper tragende Schwenkachse als U-förmig gebogene Drahtfeder ausgebildet, über die der Ventilkörper in die Schließstellung vorgespannt ist. Die freien Enden der Feder sind an unterschiedlichen Stellen rechtwinklig nach innen umgebogen und in versetzt zueinander angeordneten Bohrungen im Gehäuse festgelegt. Aufgrund der unterschiedlichen Schenkellängen der Feder bewirkt das Verschwenken des Ventilkörpers ein Spannen der Feder, so dass diese den Ventilkörper in Richtung auf die Schließstellung belastet. Die Ausbildung der Bohrungen zur Aufnahme der freien Enden der Feder macht das Einhalten enger Toleranzwerte erforderlich, wodurch die Herstellung arbeitsaufwendig und somit teuer wird. Darüber hinaus ist diese bekannte Ventileinrichtung nur schwer zu reinigen.

Davon ausgehend liegt der Erfindung die **Aufgabe** zugrunde, eine Ventileinrichtung für medizinische Instrumente der eingangs genannten Art so auszugestalten, dass diese bei einfachem und kostengünstigem Aufbau gut zu reinigen ist.

Die **Lösung** dieser Aufgabenstellung ist erfindungsgemäß dadurch gekennzeichnet, dass die Schwenkachse mehrteilig ausgebildet ist, wobei die einzelnen Teilstücke der Schwenkachse über wendelförmig ausgebildete Führungsbahnen miteinander verbindbar sind.

Durch die erfindungsgemäße Ausgestaltung der Schwenkachse als mehrteilige Wendelstange, auf der der Ventilkörper verschwenkbar gelagert ist, ergibt sich eine einfach zu fertigende sowie schnell und unkompliziert zu montierende Bauweise, die sich zu Reinigungszwecken problemlos und schnell demontieren lässt. Darüber hinaus gewährleisten die ineinander schraubbaren Wendelabschnitte der einzelnen Teilstücke der Schwenkachse ein leichtgängiges Verschwenken des Ventilkörpers.

Gemäß einer praktischen Ausführungsform der Erfindung ist ein Teilstück der Schwenkachse ausschließlich um seine Längsachse verschwenkbar und nicht translatorisch verlagerbar im Gehäuse gelagert und der Ventilkörper vorzugsweise auf diesem ausschließlich um seine Längsachse verschwenkbaren Teilstück der Schwenkachse drehfest gelagert. Das Ausführen einer radialen Schwenkbewegung dieses Teilstücks der Schwenkachse ausschließlich um seine Achse gewährleistet ein klemmfreies Verschwenken des Ventilkörpers zwischen der Offensteflung und der Schließstellung und eine immer lagegenaue Anlage des Ventilkörpers an den Dichtflächen des Gehäuses.

Mit einer bevorzugten Ausführungsform der Erfindung wird vorgeschlagen, dass die Schwenkachse aus zwei Teilstücken besteht, wobei ein Teilstück ausschließlich um seine Längsachse verschwenkbar und nicht translatorisch verlagerbar im Gehäuse gelagert ist und das andere Teilstück ausschließlich in Längsrichtung des Teilstücks axial verschiebbar und nicht schwenkbar im Gehäuse gelagert ist. Durch die Ausbildung der Schwenkachse aus nur zwei Teilstücken, die über wendelförmig ausgebildete Führungsbahnen miteinander verbindbar sind, wird die Anzahl der Bauteile auf ein Minimum reduziert, was sich wiederum positiv auf die Montage und Demontage der Ventileinrichtung auswirkt.

Gemäß einer alternativen Ausführungsform der Erfindung wird vorgeschlagen, dass die Schwenkachse zumindest teilweise in einer Achshülse gelagert ist. Die Verwendung dieser Achshülse ermöglicht einerseits eine seitliche Führung der mehrteiligen Schwenkachse und erleichtert darüber hinaus die Montage und Demontage der Ventileinrichtung durch die Ausbildung vormontierbarer Baugruppen.

Überdies ist bei dieser Ausführungsform die Schmierung zwischen dem Teilstück, das ausschließlich in seiner Längsrichtung axial verschiebbar und nicht verschwenkbar gelagert ist, und dem Teilstück, das ausschließlich um seine Längsachse schwenkbar und nicht translatorisch verlagerbar ist, gegenüber dem Fluidkanal gekapselt.

Bei der Verwendung der Achshülse zur Lagerung der Schwenkachse ist es vorteilhaft den Ventilkörper auf der Achshülse zu lagern, um die Montage und Demontage der Baugruppen zu vereinfachen. Zur Übertragung der Drehbewegung des ausschließlich um seine Längsachse verschwenkbaren Teilstücks der Schwenkachse auf den auf der Achshülse gelagerten Ventilkörper wird erfindungsgemäß vorgeschlagen, dass der Ventilkörper und die Achshülse im Wesentlichen formschlüssig miteinander verbunden sind und das ausschließlich um seine Längsachse verschwenkbare Teilstück der Schwenkachse und die Achshülse stoffschlüssig miteinander verbunden sind.

Alternativ zu diesen bevorzugten Verbindungsarten zur Übertragung der Drehbewegung des ausschließlich um seine Längsachse verschwenkbaren Teilstücks der Schwenkachse auf den auf der Achshülse gelagerten Ventilkörper sind selbstverständlich auch alle anderen Kombinationen von Verbindungsarten, wie formschlüssig, stoffschlüssig und/oder kraftschlüssig, anwendbar.

Um beispielsweise bei der Verwendung einer erfindungsgemäßen Ventileinrichtung in einem Trokar zu vermeiden, dass empfindliche Instrumentenspitzen dadurch beschädigt werden, dass sie beim Einführen des Instruments in den Instrumentenkanal den Ventilkörper aufstoßen müssen und damit bei der Entnahme von Gewebestücken die Gewebeprobe durch die Ventilklappe nicht beschädigt wird bzw. nicht von der Fasszange abgestreift wird, wird mit der Erfindung weiterhin vorgeschlagen, dass der Ventilköper zusätzlich über einen manuell betätigbaren Mechanismus in die Offenstellung verlagerbar ist. Mittels dieses Mechanismus besteht nunmehr die Möglichkeit, alternativ zum Aufstoßen des Ventilkörpers mit dem in den Instrumentenkanal eingeführten Instrument, den Ventilkörper von außen manuell zu öffnen, um so beispielsweise den Kontakt der Instrumentenspitze und/oder der Gewebeprobe mit dem Ventilkörper zu verhindern.

Gemäß einer praktischen Ausführungsform der Erfindung ist dieser manuell betätigbare Mechanismus als mit dem ausschließlich in Längsrichtung axial verschiebbar gelagerten und nicht verschwenkbaren Teilstück der Schwenkachse in Wirkverbindung stehender Druckknopf ausgebildet. Der Druckknopf ist dabei vorzugsweise über mindestens ein Federelement gegenüber dem axial verschiebbaren und nicht verschwenkbaren Teilstück der Schwenkachse vorgespannt, um einerseits ein versehentliches Öffnen des Ventilkörpers zu verhindern und andererseits den Ventilkörper in die Schließstellung vorzuspannen.

Die Vorspannung des mindestens einen Federelements ist dabei vorteilhafterweise über eine im Bereich des manuell betätigbaren Mechanismus auf eine Lagerbuchse des Gehäuses aufschraubbare Spannmutter einstellbar.

Um weiterhin zu gewährleisten, dass das ausschließlich in Längsrichtung axial verschiebbar gelagerte Teilstück der Schwenkachse keine Schwenkbewegung um seine Achse ausführt, ist dieses Teilstück der Schwenkachse über eine Führungsstift gegen Verdrehen gesichert, wobei der Führungsstift im Bereich des manuell betätigbaren Mechanismus, beispielsweise in der Spannmutter, in einer Ausnehmung des axial verschiebbaren Teilstücks der Schwenkachse gelagert ist.

Schließlich wird mit der Erfindung vorgeschlagen, dass das den Ventilkörper sowie die Schwenkachse tragende Gehäuseteil eine separate Ventilträgereinheit bildet. Diese als separates Bauteil ausgebildete Ventilträgereinheit ermöglicht es, die erfindungsgemäße Ventileinrichtung auch für andere medizinische Instrumente und Einrichtungen zu verwenden, beispielsweise als Zwischenelement zum Verbinden von zwei Instrumentenkanälen.

Weitere Merkmale und Vorteile der Erfindung ergeben sich anhand der zugehörigen Zeichnung, in der zwei Ausführungsbeispiel einer erfindungsgemäßen Ventileinrichtung für medizinische Instrumente nur beispielhaft dargestellt sind, ohne die Erfindung auf diese Ausführungsbeispiele zu beschränken. In der Zeichnung zeigt:
- Fig. 1: eine schematische Seitenansicht einer an einer Rohrleitung angeordneten Ventileinrichtung für medizinische Instrumente;
- Fig. 2: eine teilweise geschnittene perspektivische Ansicht der Ventileinrichtung gemäß Fig. 1 ohne angeschlossene Rohrleitung, eine erste erfindungsgemäße Ausführungsform darstellend;
- Fig. 3: eine teilweise geschnittene Vorderansicht der Darstellung gemäß Fig. 2;
- Fig. 4: eine perspektivische Ansicht der Schwenkachse mit darauf gelagertem Ventilkörper der Ventileinrichtung gemäß Fig. 1 bis 3;
- Fig. 5: eine perspektivische Ansicht der Schwenkachse gemäß Fig. 4, jedoch ohne auf der Schwenkachse gelagertem Ventilkörper;
- Fig. 6: eine perspektivische Ansicht einer zweiten Ausführungsform einer erfindungsgemäßen Ventileinrichtung für medizinische Instrumente;
- Fig. 7: eine geschnittene Vorderansicht der Darstellung gemäß Fig. 6;
- Fig. 8: eine teilweise geschnittene Vorderansicht der Schwenkachse mit darauf gelagertem Ventilkörper der Ventileinrichtung gemäß Fig. 6 und 7 und
- Fig. 9: eine perspektivische Ansicht der Achshülse sowie der Ventilklappe.

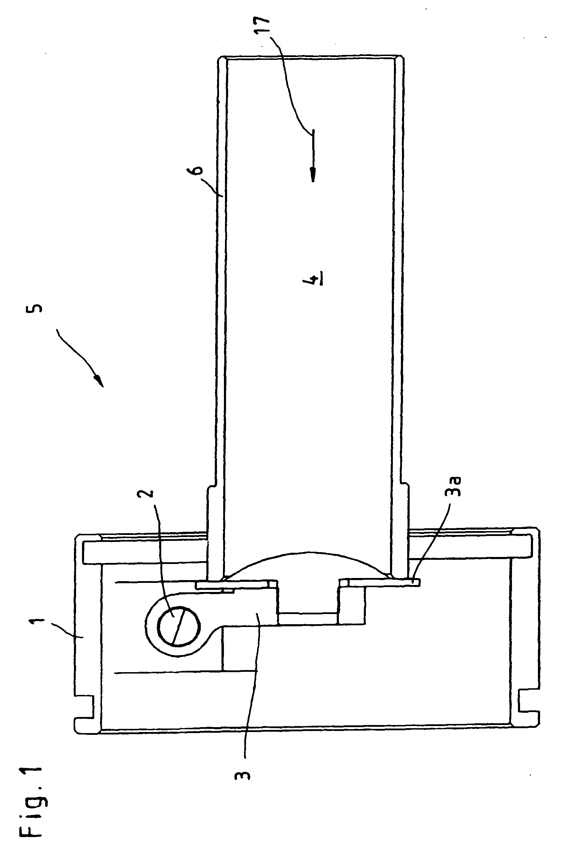

Die in den Abbildungen Fig. 1 dargestellte Ventileinrichtung für medizinische Instrumente besteht im Wesentlichen aus einem in einem Gehäuse 1 verschwenkbar auf einer Schwenkachse 2 gelagerten Ventilkörper 3, über den ein Instrumentenkanal- und/oder Strömungskanal 4 verschlossen und wieder geöffnet werden kann. Bei den dargestellten Ausführungsformen ist der Ventilkörper 3 als Ventilklappe 3 ausgebildet.

Bei der in Fig. 1 dargestellten Ausführungsform sind die Schwenkachse 2 mit dem darauf gelagerten Ventilkörper 3 sowie das die Schwenkachse 2 tragende Gehäuseteil 1 als separate Ventilträgereinheit 5 ausgebildet, die als eigenständiges Bauteil in verschiedenen medizinischen Instrumenten einsetzbar ist. Im dargestellten Fall ist an die Ventilträgereinheit 5 eine Rohrleitung 6 angekoppelt, deren Instrumenten- und/oder Strömungskanal 4 über eine Dichtfläche 3a des als Klappe ausgebildeten Ventilkörpers 3 verschließbar ist.

Insbesondere werden die dargestellten Ventileinrichtungen bei als Trokaren ausgebildeten medizinischen Instrumenten verwendet. Der hohle Instrumentenkanal 4 dient bei einem Trokar dazu, um zu Beginn der Operation einen Trokardorn aufzunehmen, mit dem eine Öffnung, beispielsweise in die Bauchdecke des Patienten, gestoßen wird. In diese Öffnung wird dann die Trokarhülse eingeschoben und der Trokardorn anschließend wieder aus dem Instrumentenkanal 4 herausgezogen. Während der nachfolgenden Operation können dann über den Instrumentenkanal 4 die unterschiedlichsten endoskopischen Instrumente in das Operationsgebiet eingeführt werden. Da es bei endoskopischen Operationen des Bauchraums üblich ist, den Bauchraum des Patienten zur Erweiterung des Operationsraums und Ausbildung eines Pneumoperitoneums mit Gas zu befüllen, ist der hohle Instrumentenkanal 4 des Trokars über den Ventilkörper 3 verschließbar, um das Austreten des Gases beim Herausziehen eines Instruments aus dem Instrumentenkanal 4 zu verhindern.

Selbstverständlich ist es auch möglich, den hohlen Kanal als Strömungskanal 4 für ein flüssiges oder gasförmiges Medium auszubilden, wobei der Ventilkörper 3 dann dazu dient, den Durchtritt dieses Mediums zu verhindern oder aber freizugeben.

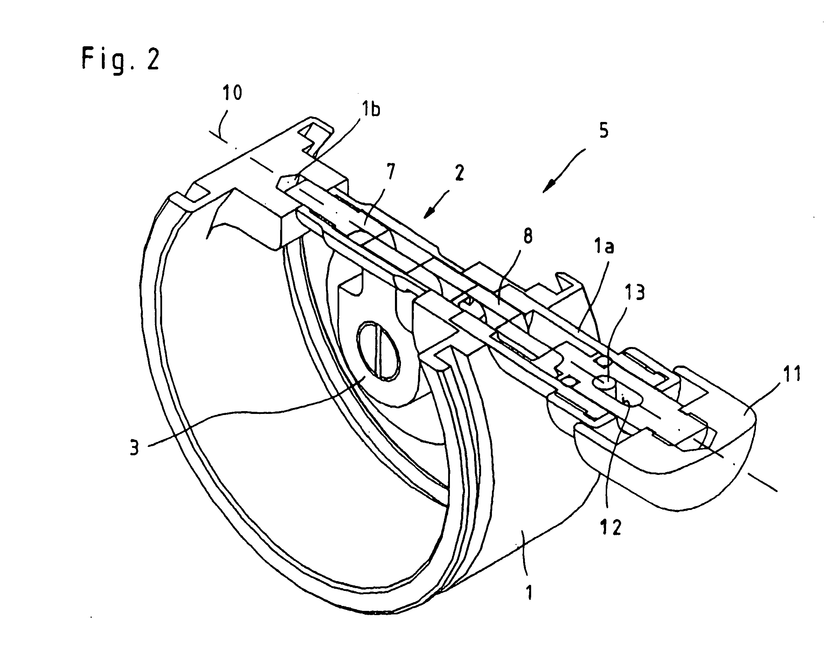

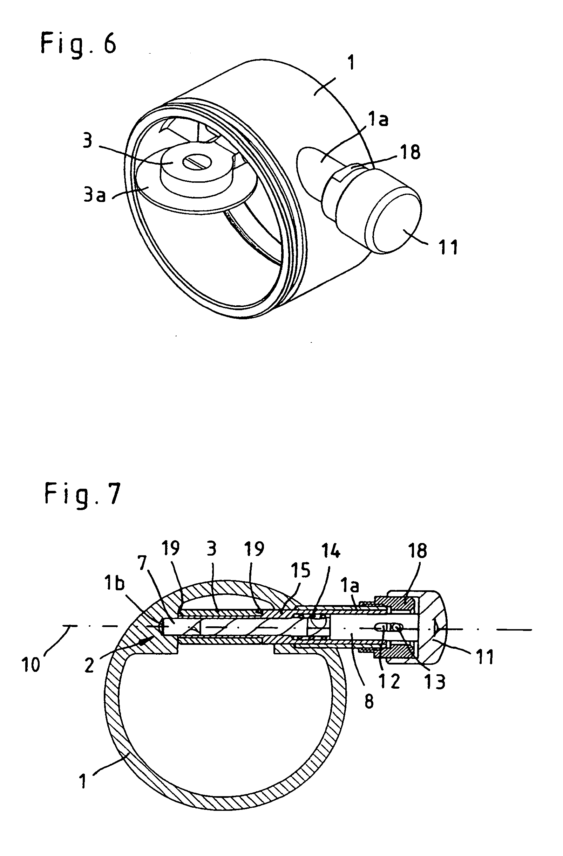

Der genaue Aufbau der Ventilträgereinheit 5 ist den Abbildungen Fig. 2 bis 8 zu entnehmen, die insbesondere den Aufbau und die Lagerung der Schwenkachse 2 im Gehäuse 1 verdeutlichen, wobei die Abbildungen Fig. 2 bis 4 die Ausgestaltung gemäß einer ersten Ausführungsform und die Abbildungen Fig. 6 bis 8 die Ausgestaltung gemäß einer zweiten Ausführungsform darstellen.

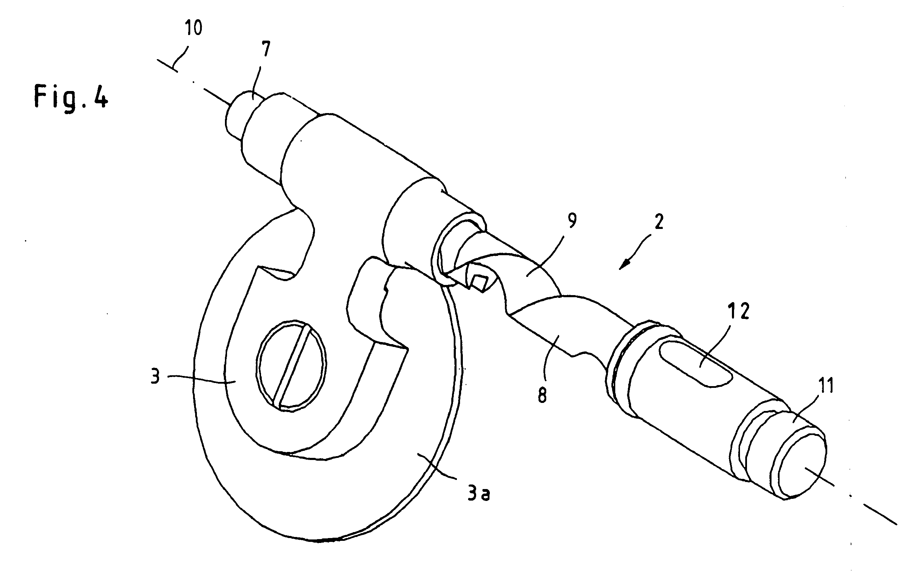

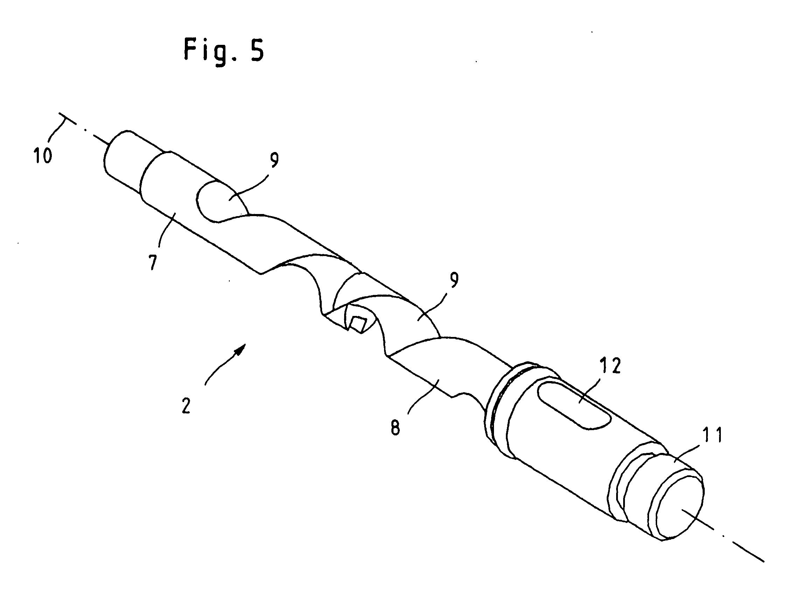

Wie aus den Abbildungen Fig. 2, 3 und Fig. 7, 8 sowie den Detailansichten gemäß Fig. 4 und 5 ersichtlich, besteht die Schwenkachse 2 bei der dargestellten Ausführungsform aus zwei Teilstücken 7 und 8, die über in den Teilstücken 7, 8 ausgebildete wendelförmige Führungsbahnen 9 miteinander verbindbar sind. Die beiden Teilstücke 7 und 8 der Schwenkachse 2 sind dabei so im Gehäuse 1 gelagert, dass das den Ventilkörper 3 tragende Teilstück 7 ausschließlich um seine Längsachse 10 verschwenkbar und nicht translatorisch verlagerbar im Gehäuse 1 gelagert ist und das zweite Teilstück 8 ausschließlich in Längsrichtung des Teilstücks 8 axial verschiebbar und nicht verschwenkbar im Gehäuse 1 gelagert ist.

Da bei Instrumenten mit besonders scharfen und/oder empfindlichen Spitzen die Gefahr besteht, dass diese Spitzen beim Aufstoßen des Ventilkörpers 3 stumpf werden oder sogar beschädigt werden und damit bei der Entnahme von Gewebestücken durch die Ventilklappe die Gewebeprobe nicht beschädigt wird bzw. nicht von der Fasszange abgestreift wird, besteht die Möglichkeit, den Ventilkörper 3 über einen manuell betätigbaren Mechanismus zu öffnen.

Bei der dargestellten Ausführungsform ist dieser Mechanismus als mit dem ausschließlich in Längsrichtung axial verschiebbar gelagerten und nicht verschwenkbaren Teilstück 8 der Schwenkachse 2 in Wirkverbindung stehender Druckknopf 11 ausgebildet, der am freien Ende einer fest mit dem Gehäuse verbundenen Lagerbuchse 1 a angeordnet ist. Um zu gewährleisten, dass eine Betätigung des Druckknopfes 11 eine ausschließlich axiale Verschiebung des Teilstücks 8 der Schwenkachse 2 bewirkt, nicht aber ein radiales Verdrehen des Teilstücks 8 aufgrund des Ineinanderlaufens der wendelförmigen Führungsbahnen 9 der beiden Teilstücke 7 und 8 der Schwenkachse 2, ist im Bereich des Druckknopfes 11 ein in einer Ausnehmung 12 des Teilstücks 8 gelagerter Führungsstift 13 vorgesehen, der das Teilstück 8 gegen radiales Verdrehen blockiert.

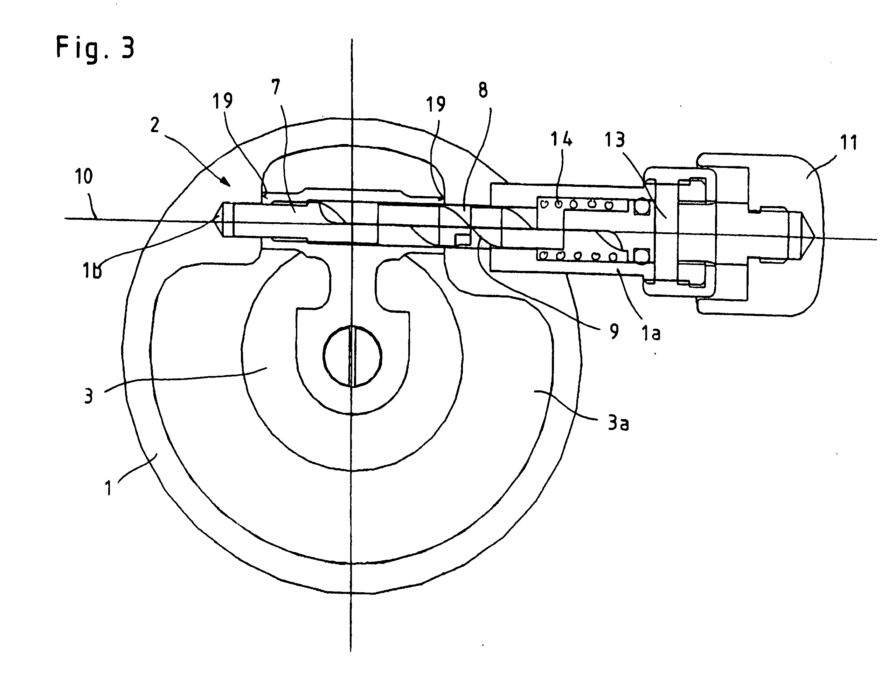

Wie weiterhin aus Fig. 3 ersichtlich, ist der Druckknopf 11 über mindestens ein Federelement 14 gegenüber dem axial verschiebbaren Teilstück 8 der Schwenkachse 2 vorgespannt, um einerseits ein versehentliches Öffnen des Ventilkörpers 3 zu verhindern und andererseits den Ventilkörper 3 in die Schließstellung vorzuspannen.

Der wesentliche Unterschied zwischen der in den Abbildungen Fig. 6 bis 8 dargestellten zweiten Ausführungsform einer Ventileinrichtung für medizinische Instrumente und der in den Abbildungen Fig. 2 bis 4 dargestellten ersten Ausführungsform besteht darin, dass die beiden die Schwenkachse 2 bildenden Teilstücke 7 und 8 bei dieser zweiten Ausgestaltungsform zumindest teilweise in einer die Schwenkachse 2 koaxial umgebenden Achshülse 15 gelagert sind. Die Verwendung dieser Achshülse 15 ermöglicht einerseits eine seitliche Führung der mehrteiligen Schwenkachse 2 und erleichtert darüber hinaus die Montage und Demontage der Ventileinrichtung durch die Ausbildung vormontierbarer Baugruppen, wie dies nachfolgend noch genauer beschrieben wird..

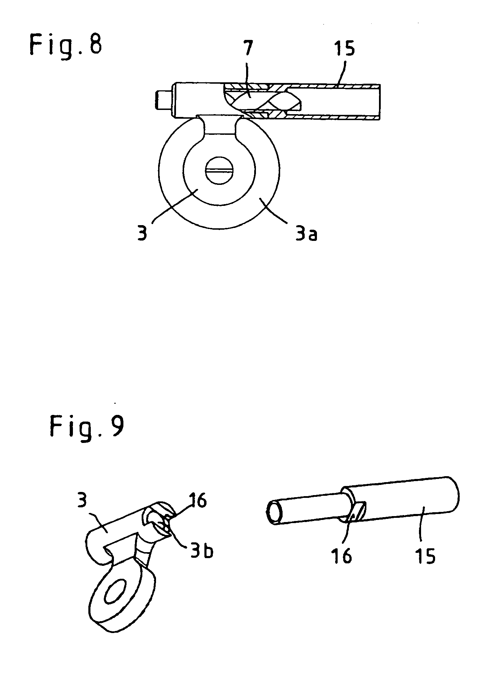

Wie insbesondere aus Fig. 7 und 8 ersichtlich, ist bei dieser Ausgestaltungsform der Ventileinrichtung der auch hier als Ventilklappe 3 ausgebildete Ventilkörper 3 nicht direkt auf dem ausschließlich um seine Längsachse 10 verschwenkbaren Teilstück 7 der Schwenkachse 2 gelagert, sondern auf der dieses Teilstück 7 koaxial umgebenden Achshülse 15.

Um zu gewährleisten, dass das Verschwenken des Teilstücks 7 der Schwenkachse 2 auch ein Verschwenken des Ventilkörpers 3 bewirkt, sind bei dieser Ausführungsform der Ausbildung und Lagerung der Schwenkachse einerseits das ausschließlich um seine Längsachse 10 verschwenkbare Teilstück 7 der Schwenkachse 2 und die Achshülse 15 stoffschlüssig miteinander verbunden und andererseits der Ventilkörper 3 und die Achshülse 15 formschlüssig miteinander verbunden. Wie aus Fig. 9 ersichtlich, kann der Formschluss zwischen dem Ventilkörper 3 und der Achshülse 15 beispielsweise dadurch bewirkt werden, dass an der Achshülse 15 einerseits und dem auf der Achshülse 15 zu lagernden Ventilkörper 3 andererseits miteinander korrespondierende Anlagefläche 16, beispielsweise parallele Abflachungen, ausgebildet sind die sicherstellen, dass das Verschwenken des einem Bauteils 3 oder 15 immer auch ein Verschwenken des anderen Bauteils 15 oder 3 bewirkt.

Alternativ zu diesen bevorzugten Verbindungsarten zur Übertragung der Drehbewegung des ausschließlich um seine Längsachse verschwenkbaren Teilstücks 7 der Schwenkachse 2 auf den auf der Achshülse 15 gelagerten Ventilkörper 3 sind selbstverständlich auch alle anderen Kombinationen von Verbindungsarten, wie formschlüssig, stoffschlüssig und/oder kraftschlüssig, anwendbar.

Die in den Abbildungen Fig. 1 bis 9 dargestellten Ventileinrichtungen für medizinische Instrumente arbeiten wie folgt:

Ausgehend von der in den Abbildungen Fig. 1 bis 3 dargestellten Schließstellung des Ventilkörpers 3, in der die Dichtfläche 3a des Ventilkörpers 3 den Instrumenten- und/oder Strömungskanal 4 fluiddicht abdichtet, ist der auf der im Gehäuse 1 gelagerten Schwenkachse 2 angeordnete Ventilkörper 3 um die Längsachse 10 der Schwenkachse 2 verschwenkbar in eine den Instrumenten- und/oder Strömungskanal 4 freigebende Offenstellung überführbar, wie diese in Fig. 6 dargestellt ist. Hierbei ist es für die Verlagerung des Ventilkörpers 3 unerheblich, ob dieser wie bei der ersten Ausführungsform direkt auf der Schwenkachse 2 gelagert ist, oder aber der Ventilkörper 3 indirekt, nämlich unter Zwischenschaltung der Achshülse 15, auf der Schwenkachse 2 gelagert ist.

Das Überführen des Ventilkörpers 3 in die Offenstellung kann dadurch erfolgen, dass ein medizinisches Instrument in Richtung des Pfeils 17 in Fig. 1 in den Instrumenten- und/oder Strömungskanal 4 eingeführt wird und dieses eingeführte medizinische Instrument den Ventilkörper 3 in die Offenstellung drückt.

Gleichwirkend mit dieser mechanischen Öffnung der Ventileinrichtung über ein in den Instrumenten- und/oder Strömungskanal 4 eingeführtes medizinisches Instrument kann das Öffnen durch eine in Richtung des Pfeils 17 erfolgende Fluidströmung erfolgen die groß genug ist, um die Zuhaltekraft des Federelements 14 zu überwinden.

Dieses durch eine unmittelbar auf den Ventilkörper 3 wirkende Druckkraft induzierte Verschwenken des Ventilkörpers 3 bewirkt ein Verdrehen des ausschließlich um die Längsachse 10 verdrehbaren Teilstücks 7 der Schwenkachse 2, wobei durch das Ineinandergreifen der wendelförmigen Führungsbahnen 9 der beiden Teilstücke 7, 8 das Verdrehen des Teilstücks 7 zwangsläufig eine Axialverschiebung des ausschließlich axial verschiebbaren Teilstücks 8 bewirkt, und zwar in diesem Fall eine Verschiebung nach links gemäßen den Darstellungen in Fig. 2, 3 und 7.

Zusätzlich zu der Verlagerung des Ventilkörpers 3 in die Offenstellung über direkt auf den Ventilkörper 3 einwirkende Kräfte ist es möglich, die Ventileinrichtung über den als Druckknopf 11 ausgebildeten manuell betätigbaren Öffnungsmechanismus zu betätigen.

Das Eindrücken des Druckknopfes 11 gegen die Federkraft des Federelements 14 bewirkt aufgrund der Sperre durch den Führungsstift 13 ein rein axiales Verschieben des Teilstücks 8 der Schwenkachse 2 in Richtung des Teilstücks 7 der Schwenkachse 2. Bei der in den Abbildungen Fig. 6 und 7 dargestellten zweiten Ausführungsform ist die Vorspannkraft des Federelements 14 über eine auf die Lagerbuchse 1a aufschraubbare Spannmutter 18 einstellbar, so dass bei dieser Ausgestaltungsform die Möglichkeit besteht, bei Undichtigkeiten den Anpressdruck der Dichtung anzupassen. Der Führungsstift 13 zum Sperren des rein axial verschiebbaren Teilstücks 8 der Schwenkachse 2 ist bei der dargestellten zweiten Ausführungsform in der Spannmutter 18 gelagert.

Aufgrund der Anordnung des Ventilkörpers 3 zwischen zwei Anschlagflächen 19 im Gehäuse 1 bei der ersten Ausführungsform und einer Anschlagfläche 19 am Gehäuse 1 und einer Anschlagfläche 19 an der Achshülse 15 bei der zweiten Ausführungsform kann das Teilstück 7 der Schwenkachse 2 dieser Axialverschiebung des Teilstücks 8 nicht folgen, weshalb das Andrücken des Teilstücks 8 gegen das Teilstück 7 aufgrund der ineinander greifenden Führungsbahnen 9 und der längsfesten Verbindung zwischen dem Ventilkörper 3 und dem Teilstück 7 der Schwenkachse 2 zu einem Verschwenken des Teilstücks 7 sowie des direkt oder indirekt auf diesem Teilstück 7 gelagerten Ventilkörpers 3 um die Längsachse 10 der Schwenkachse führt.

Sobald keine Druckkraft mehr auf den Druckknopf 11 ausgeübt wird, bewirkt das Federelement 14 ein Verschieben des Teilstücks 8 in die entgegengesetzte Richtung, das heißt fort vom Teilstück 7 der Schwenkachse 2. Diese Verschiebung des axial verschiebbaren Teilstücks 8 bewirkt dann zwangsläufig über die Kopplung der wendelförmigen Führungsbahnen 9 ein Verschwenken des Teilstücks 7 und somit des Ventilkörpers 3 zurück in die Schließstellung.

Die Montage der in den Abbildungen Fig. 6 bis 9 dargestellten zweiten Ausführungsform einer Ventileinrichtungen für medizinische Instrumente geschieht wie folgt:

Vorteilhaft an der Ventileinrichtung gemäß der zweiten Ausführungsform ist, dass einzelne Bauteile zur vorgefertigten Baugruppen zusammensetzbar sind, wodurch die Montage und Demontage deutlich vereinfacht werden kann.

Vor der Endmontage der kompletten Ventileinrichtung wird zunächst das ausschließlich um die Längsachse 10 verdrehbare Teilstück 7 der Schwenkachse 2 in die Achshülse 15 eingesetzt und mit dieser, beispielsweise durch Verschweißen, stoffschlüsssig verbunden. Anschließend wird der Ventilkörper 3 mit der als Flachdichtung ausgebildeten Dichtfläche 3a bestückt. Zur Komplettierung der Schwenkachse 2 werden nun das als Druckfeder ausgebildete Federelement 14 sowie das ausschließlich axial verschiebbare Teilstück 8 der Schwenkachse 2 in die Achshülse 15 in die bereits mit dem Teilstück 7 der Schwenkachse 2 versehene Achshülse 15 eingesetzt.

Anschließend wird der vorgefertigte Ventilkörper 3 so in das Ventilgehäuse 1 eingesetzt, dass die Bohrung 3b zur Aufnahme der Achshülse 15 einerseits mit der Lagerbuchse 1 a und andererseits mit einer Lagerbohrung 1b im Gehäuse 1 fluchtet. In die Bohrung 3b des Ventilkörpers 3 kann nunmehr über das freie Ende der Lagerbuchse 1a die Achshülse 15 mitsamt der vorgefertigten Schwenkachse 2 eingeschoben werden, bis das freie Ende des Teilstücks 7 Aufnahme in der Lagerbohrung 1 b findet.

Zur Einstellung der Vorspannung des Federelements 14 wird jetzt die Spannmutter 18 auf die Lagerbuchse 1 a aufgeschraubt und der Druckknopf 11 auf das das freie Ende der Lagerbuchse 1a überragende freie Ende des Teilstücks 8 der Schwenkachse 2 aufgesetzt, über den Druck auf das Federelement 14 ausgeübt werden kann. Nach dem Einstellen der gewünschten Vorspannung des Federelements 14, was während der Montage und insbesondere auch nach der Montage, beispielsweise zur Beseitigung von Undichtigkeiten, erfolgen kann, wird der das Teilstück 8 gegen Verdrehen sichernde Führungsstift 13 in die Spannmutter 18 und die Ausnehmung 12 des Teilstücks 8 der Schwenkachse 2 eingesetzt.

Eine solchermaßen ausgebildete Ventileinrichtung zeichnet sich durch ihren einfachen und aus nur wenigen Bauteilen bestehenden Aufbau aus, der bei zuverlässiger Dichtleistung eine einfache und schnelle Montage und Demontage der Ventileinrichtung, beispielsweise zu Reinigungszwecken, ermöglicht.

### Bezugszeichenliste

- 1: Gehäuse
- 1a: Lagerbuchse
- 1b: Lagerbohrung
- 2: Schwenkachse
- 3: Ventilkörper / Ventilklappe
- 3a: Dichtfläche
- 3b: Bohrung
- 4: Instrumenten- / Strömungskanal
- 5: Ventilträgereinheit
- 6: Rohrleitung
- 7: Teilstück (radial verschwenkbar)
- 8: Teilstück (axial verschiebbar)
- 9: Führungsbahn
- 10: Längsachse
- 11: Druckknopf
- 12: Ausnehmung
- 13: Führungsstift
- 14: Federelement
- 15: Achshülse
- 16: Anlagefläche
- 17: Strömungs- / Einführrichtung
- 18: Spannmutter
- 19: Anschlagfläche

## Patentansprüche

1. Ventileinrichtung für medizinische Instrumente, insbesondere Trokare, mit einem als Klappe ausgebildeten Ventilkörper (3) zum Öffnen und Schließen eines Instrumenten- und/oder Strömungskanals (4), der auf einer in einem Gehäuse (1) gelagerten Schwenkachse (2) angeordnet ist,
**dadurch gekennzeichnet,**
**dass** die Schwenkachse (2) mehrteilig ausgebildet ist, wobei die einzelnen Teilstücke (7, 8) der Schwenkachse (2) über wendelförmig ausgebildete Führungsbahnen (9) miteinander verbindbar sind.

2. Ventileinrichtung nach Anspruch 1, **dadurch gekennzeichnet, dass** ein Teilstück (7) der Schwenkachse (2) ausschließlich um seine Längsachse (10) verschwenkbar im Gehäuse (1) gelagert ist.

3. Ventileinrichtung nach Anspruch 2, **dadurch gekennzeichnet, dass** die Schwenkachse (2) aus zwei Teilstücken (7, 8) besteht, wobei ein Teilstück (7) ausschließlich um seine Längsachse (10) verschwenkbar im Gehäuse (1) gelagert ist und das andere Teilstück (8) ausschließlich in Längsrichtung des Teilstücks (8) axial verschiebbar im Gehäuse (1) gelagert ist.

4. Ventileinrichtung nach Anspruch 2 oder 3, **dadurch gekennzeichnet, dass** der Ventilkörper (3) auf dem ausschließlich um seine Längsachse (10) verschwenkbaren Teilstück (7) der Schwenkachse (2) gelagert ist.

5. Ventileinrichtung nach einem der Ansprüche 1 bis 4, **dadurch gekennzeichnet, dass** die Schwenkachse (2) zumindest teilweise in einer Achshülse (15) gelagert ist.

6. Ventileinrichtung nach Anspruch 5, **dadurch gekennzeichnet, dass** der Ventilkörper (3) auf der Achshülse (15) gelagert ist.

7. Ventileinrichtung nach Anspruch 6, **dadurch gekennzeichnet, dass** der Ventilkörper (3) und die Achshülse (15) im Wesentlichen formschlüssig miteinander verbunden sind.

8. Ventileinrichtung nach Anspruch 6 oder 7 in Abhängigkeit von Anspruch 2, **dadurch gekennzeichnet, dass** das ausschließlich um seine Längsachse (10) verschwenkbare Teilstück (7) der Schwenkachse (2) und die Achshülse (15) stoffschlüssig miteinander verbunden sind.

9. Ventileinrichtung nach einem der Ansprüche 1 bis 8, **dadurch gekennzeichnet, dass** der auf der Schwenkachse (2) gelagerte Ventilkörper (3) über einen manuell betätigbaren Mechanismus in die Offenstellung verlagerbar ist.

10. Ventileinrichtung nach Anspruch 3 und 9, **dadurch gekennzeichnet, dass** der manuell betätigbare Mechanismus als mit dem ausschließlich in Längsrichtung axial verschiebbar gelagerten Teilstück (8) der Schwenkachse (2) in Wirkverbindung stehender Druckknopf (11) ausgebildet ist.

11. Ventileinrichtung nach Anspruch 10, **dadurch gekennzeichnet, dass** der Druckknopf (11) über mindestens ein Federelement (14) gegenüber dem axial verschiebbaren Teilstück (8) der Schwenkachse (2) vorgespannt ist.

12. Ventileinrichtung nach Anspruch 11, **dadurch gekennzeichnet, dass** der Ventilkörper (3) über das mindestens eine Federelement (14) in Richtung der Schließstellung vorgespannt ist.

13. Ventileinrichtung nach Anspruch 11 oder 12, **dadurch gekennzeichnet, dass** die Vorspannung des mindestens einen Federelements (14) über eine im Bereich des manuell betätigbaren Mechanismus auf eine Lagerbuchse (1a) des Gehäuses (1) aufschraubbare Spannmutter (18) einstellbar ist.

14. Ventileinrichtung nach Anspruch 3 **dadurch gekennzeichnet, dass** das ausschließlich in Längsrichtung axial verschiebbar gelagerte Teilstück (8) der Schwenkachse (2) über einen Führungsstift (13) gegen Verdrehen gesichert ist.

15. Ventileinrichtung nach Anspruch 14, **dadurch gekennzeichnet, dass** der Führungsstift (13) im Bereich des manuell betätigbaren Mechanismus in einer Ausnehmung (12) des axial verschiebbaren Teilstücks (8) der Schwenkachse (2) gelagert ist.

16. Ventileinrichtung nach den Ansprüchen 13 bis 15, **dadurch gekennzeichnet, dass** der Führungsstift (13) in der Spannmutter (18) gelagert ist.

17. Ventileinrichtung nach einem der Ansprüche 1 bis 16, **dadurch gekennzeichnet, dass** das den Ventilkörper (3) sowie die Schwenkachse (2) tragende Gehäuseteil (1) eine separate Ventilträgereinheit (5) bildet.

## Claims

1. Valve device for medical instruments, in particular trocars, with a valve body (3) which is designed as a flap for opening and closing an instrument channel and/or flow channel (4) and which is arranged on a pivot shaft (2) mounted in a housing (1), **characterized in that** the pivot shaft (2) has a multi-part design, in which the individual constituent parts (7, 8) of the pivot shaft (2) can be connected to each other via helically shaped guide tracks (9).

2. Valve device according to Claim 1, **characterized in that** a constituent part (7) of the pivot shaft (2) is mounted in the housing (1) to be exclusively pivotable about its longitudinal axis (10).

3. Valve device according to Claim 2, **characterized in that** the pivot shaft (2) consists of two constituent part (7, 8), one constituent part (7) being mounted in the housing (1) to be exclusively pivotable about its longitudinal axis (10), and the other constituent part (8) being mounted in the housing (1) to be exclusively axially displaceable in the longitudinal direction of the constituent part (8).

4. Valve device according to Claim 2 or 3, **characterized in that** the valve body (3) is mounted on the constituent part (7) of the pivot shaft (2) exclusively pivotable about its longitudinal axis (10).

5. Valve device according to one of Claims 1 to 4, **characterized in that** the pivot shaft (2) is mounted at least partially in a shaft sleeve (15).

6. Valve device according to Claim 5, **characterized in that** the valve body (3) is mounted on the shaft sleeve (15).

7. Valve device according to Claim 6, **characterized in that** the valve body (3) and the shaft sleeve (15) are connected to each other substantially with a form fit.

8. Valve device according to Claim 6 or 7 as dependent on Claim 2, **characterized in that** the constituent part (7) of the pivot shaft (2), exclusively pivotable about its longitudinal axis (10), and the shaft sleeve (15) are connected to each other with a cohesive material fit.

9. Valve device according to one of Claims 1 to 8, **characterized in that** the valve body (3) mounted on the pivot shaft (2) can be moved into the open position via a manually actuated mechanism.

10. Valve device according to Claims 3 and 9, **characterized in that** the manually actuated mechanism is designed as a push-button (11) that is operatively connected to the constituent part (8) of the pivot shaft (2) mounted exclusively axially displaceably in the longitudinal direction.

11. Valve device according to Claim 10, **characterized in that** the push-button (11) is pretensioned by at least one spring element (14) with respect to the axially displaceable constituent part (8) of the pivot shaft (2).

12. Valve device according to Claim 11, **characterized in that** the valve body (3) is pretensioned by the at least one spring element (14) in the direction of the closed position.

13. Valve device according to Claim 11 or 12, **characterized in that** the pretensioning of the at least one spring element (14) can be adjusted by means of a tensioning nut (18) that can be screwed onto a bearing bushing (1a) of the housing (1) in the area of the manually actuated mechanism.

14. Valve device according to Claim 3, **characterized in that** the constituent part (8) of the pivot shaft (2) mounted to be exclusively axially displaceable in the longitudinal direction is secured against rotation by a guide pin (13).

15. Valve device according to Claim 14, **characterized in that** the guide pin (13), in the area of the manually actuated mechanism, is mounted in a recess (12) of the axially displaceable constituent part (8) of the pivot shaft (2).

16. Valve device according to Claims 13 to 15, **characterized in that** the guide pin (13) is mounted in the tensioning nut (18).

17. Valve device according to one of Claims 1 to 16, **characterized in that** the housing part (1) carrying the valve body (3) and the pivot shaft (2) forms a separate valve carrier unit (5).

## Revendications

1. Dispositif de soupape pour instruments médicaux, notamment des trocarts, comprenant un corps d'obturation de soupape (3) réalisé sous forme de clapet, qui est destiné à ouvrir et fermer un canal (4) d'instrument et/ou d'écoulement, et est agencé sur un axe de pivotement (2) monté dans un carter (1),
**caractérisé en ce que** l'axe de pivotement (2) est d'une configuration en plusieurs parties, les parties constitutives individuelles (7, 8) de l'axe de pivotement (2) pouvant être reliées l'une à l'autre par l'intermédiaire de voies de guidage (9) réalisées en forme d'hélice.

2. Dispositif de soupape selon la revendication 1, **caractérisé en ce qu'**une partie constitutive (7) de l'axe de pivotement (2) est montée dans le carter (1) en pouvant exclusivement pivoter autour de son axe longitudinal (10).

3. Dispositif de soupape selon la revendication 2, **caractérisé en ce que** l'axe de pivotement (2) est formé de deux parties constitutives (7, 8), une partie constitutive (7) étant montée dans le carter (1) en pouvant exclusivement pivoter autour de son axe longitudinal (10), et l'autre partie constitutive (8) étant montée dans le carter (1) en pouvant exclusivement coulisser axialement dans la direction longitudinale de la partie constitutive (8).

4. Dispositif de soupape selon la revendication 2 ou 3, **caractérisé en ce que** le corps d'obturation de soupape (3) est monté sur la partie constitutive (7) de l'axe de pivotement (2), qui peut exclusivement pivoter autour de son axe longitudinal (10).

5. Dispositif de soupape selon l'une des revendications 1 à 4, **caractérisé en ce que** l'axe de pivotement (2) est monté au moins partiellement dans un fourreau d'axe (15).

6. Dispositif de soupape selon la revendication 5, **caractérisé en ce que** le corps d'obturation de soupape (3) est monté sur le fourreau d'axe (15).

7. Dispositif de soupape selon la revendication 6, **caractérisé en ce que** le corps d'obturation de soupape (3) et le fourreau d'axe (15) sont reliés essentiellement par une liaison par complémentarité de formes.

8. Dispositif de soupape selon la revendication 6 ou 7, en fonction de la revendication 2, **caractérisé en ce que** partie constitutive (7) de l'axe de pivotement (2), qui peut exclusivement pivoter autour de son axe longitudinal (10), et le fourreau d'axe (15) sont reliés par une liaison par continuité de matière.

9. Dispositif de soupape selon l'une des revendications 1 à 8, **caractérisé en ce que** le corps d'obturation de soupape (3) monté sur l'axe de pivotement (2) peut être déplacé dans la position ouverte, par l'intermédiaire d'un mécanisme pouvant être actionné manuellement.

10. Dispositif de soupape selon les revendications 3 et 9, **caractérisé en ce que** le mécanisme pouvant être actionné manuellement est réalisé sous forme de bouton poussoir (11) en interaction avec la partie constitutive (8) de l'axe de pivotement (2), qui est montée de manière à pouvoir exclusivement coulisser axialement dans la direction longitudinale.

11. Dispositif de soupape selon la revendication 10, **caractérisé en ce que** le bouton poussoir (11) est précontraint, par l'intermédiaire d'au moins un élément de ressort (14), par rapport à la partie constitutive (8) de l'axe de pivotement (2), qui peut coulisser axialement.

12. Dispositif de soupape selon la revendication 11, **caractérisé en ce que** le corps d'obturation de soupape (3) est précontraint en direction de la position fermée, par ledit au moins un élément de ressort (14).

13. Dispositif de soupape selon la revendication 11 ou 12, **caractérisé en ce que** la précontrainte dudit au moins un élément de ressort (14) peut être réglée par l'intermédiaire d'un écrou de serrage (18) pouvant être vissé sur un coussinet de palier (1a) du carter (1), dans la zone du mécanisme pouvant être actionné manuellement.

14. Dispositif de soupape selon la revendication 3, **caractérisé en ce que** la partie constitutive (8) de l'axe de pivotement (2), qui est montée de manière à pouvoir exclusivement coulisser axialement dans la direction longitudinale, est arrêtée en rotation par l'intermédiaire d'une goupille de guidage (13).

15. Dispositif de soupape selon la revendication 14, **caractérisé en ce que** la goupille de guidage (13) est montée dans la zone du mécanisme pouvant être actionné manuellement, dans un évidement (12) de la partie constitutive (8) de l'axe de pivotement (2), qui peut coulisser axialement.

16. Dispositif de soupape selon l'une des revendications 13 à 15, **caractérisé en ce que** la goupille de guidage (13) est montée dans l'écrou de serrage (18).

17. Dispositif de soupape selon l'une des revendications 1 à 16, **caractérisé en ce que** la partie de carter (1) supportant le corps d'obturation de soupape (3) ainsi que l'axe de pivotement (2), forme une unité de support de soupape (5) séparée.
